# EUROPEAN PATENT SPECIFICATION

(11) **EP 2 454 409 B1**
(45) Date of publication and mention of the grant of the patent: **18.12.2013**
(21) Application number: 09829864.9
(22) Date of filing: 13.07.2009
(51) Int. Cl.: D21C 1/00, D21C 1/02, D21C 1/04, D21C 3/00, D21C 3/02, D21C 7/00, C12P 7/10, C12P 19/02, C08H 8/00

(54) **HIGH TEMPERATURE LIGNIN SEPARATION PROCESS**
HOCHTEMPERATUR VERFAHREN ZUM AUFTRENNEN VON LIGNIN
PROCÉDÉ À HAUTE TEMPÉRATURE DE SÉPARATION DE LIGNINE

(43) Date of publication of application: 23.05.2012
(73) Proprietor: BETA RENEWABLES S.p.A., 15057 Tortona (AL) (IT)
(72) Inventor: BONANNI, Andrea, I-00148 Roma (IT); CORBELLANI, Paolo, I-15057 Tortona (Alessandria) (IT)
(74) Representative: Gerbino, Angelo
(86) International application number: PCT/IT2009/000302
(87) International publication number: WO 2011/007369

(56) References cited:
- WO-A1-00/61858
- WO-A1-2006/038863
- WO-A1-2009/031164
- WO-A2-2007/146245
- WO-A2-2009/112134
- US-A- 5 705 369
- US-A1- 2003 041 982
- US-A1- 2005 269 048
- US-A1- 2007 215 300
- US-A1- 2007 259 412
- US-A1- 2008 032 344

## Description

### Background

The efficient removal of lignin is important in the conversion of cellulose and sugars from biomass to other organic compounds, such as ethanol.

United States Patent Publication 200900381212 A1 teaches the removal of lignin from paper mill liquors by increasing the temperature of the solution to above a critical temperature where the lignin releases the water. The patent further teaches when the lignin mass being heated during treatment process reaches the critical temperature for separation, lignin solids precipitate as the mass is subsequently cooled, while released water accumulates as a supernatant.

While this process may work for paper mill liquor, lignin freed during the hydrolysis and fermentation processes of biomass conversion cannot be separated in this manner.

### Summary

Disclosed in this specification is a method for separating lignin from an aqueous mixture, comprising the steps of a) heating the mixture to a temperature above a critical temperature and b) separating the lignin solids from the mixture at a separation temperature which is at or above the critical temperature, wherein such steps have the further features disclosed in claim 1 which follows.

The separation can be done via centrifuge, filtration, elutriation or other acceptable technique, including gravity.

It is further disclosed that the mixture may contain an enzyme capable of converting cellulose to sugars, that the mixture be derived from a biomass feedstock and/or the step of heating the mixture is preceded by a steam explosion step conducted upon the biomass feedstock.

The timing of conducting the separation may be after the biomass has been at least partially hydrolyzed to sugars, after the at least partially hydrolyzed biomass has been converted to an alcohol, or after a distillation step to separate an alcohol from water.

It is further disclosed that the pH of the mixture is above 3.5 or even 7.0.

It is fiuther disclosed that the critical temperature is in the range of 60°C to 98°C, or that the lower temperature of the range is 70°C and the upper temperature is 97°C.

### Detailed description

Lignin is a product that is left over after the generation of products from biomass feedstocks. For example in the production of ethanol, the biomass feedstock can be first pretreated - typically with a steam explosion, hydrolyzed to the sugars - usually in the presence of enzymes, and the sugars then fermented to yield ethanol. The ethanol is usually removed by distillation, leaving water, lignin, yeasts, and other enzymes behind.

The separation of the lignin from the solution is extremely difficult It is believed that this is caused by the high absorbency of water by the lignin. US 20090038212 alleges that low water containing liquid solids can be recovered by first separating the lignin gel from a mixture, heating the lignin to a temperature above a critical temperature, and then cooling the lignin to cause the solids to precipitate without the water.

Attempts to utilize this process to separate lignin from an aqueous mixture comprised of water, yeast, and enzymes for the enzymatic hydrolysis of cellulose have failed. The lignin does not precipate upon cooling, and in fact, the phases are almost still inseparable after high speed centrifugation.

What has been discovered is that lignin in an aqueous mixture formed during the process of converting biomass to an alcohol can be more efficiently separated when the temperature of the mixture is maintained at or above a critical temperature.

The critical temperature related to the glass transition temperature of the lignin of the solution. It is thought that once the lignin is heated to above its glass transition temperature, it releases the absorbed water. The glass transition is dependent in part upon the lignin type, the previous heat treatment (e.g. water washing, steam explosion, and hydrolysis) and the sorbed water.

Therefore, for purposes of this specification, the critical temperature is the lowest temperature at which lignin plastic solids release the sorbed water, which is believed to be about the glass transition temperature of the particular lignin, having been treated in the previous conditions. While the glass transition will vary, the critical temperature will occur in a temperature range, bounded on the low end at 10°C below the onset of the glass transition temperature of the treated lignin and 225°C on the upper end. The upper end of 225°C is determined by the temperature considered the maximum practical temperature for operation due to the large amount of water.

A more preferred range is the onset of the glass transition temperature to 97°C. Observations have shown that higher temperatures beyond a certain temperature do not yield better separation. In the experiments, separation at 80°C and 90°C yielded very similar results. Based upon what is known at present, acceptable ranges for the critical temperature are 45°C to 97°C, 60°C to 97°C, 64°C to 97°C, 70°C to 97°C, 74°C to 97°C, 79°C to 97°C and 84°C to 97°C.

However, if the lignin has been treated in other manners, it is know that the glass transition can drop dramatically.

While theoretically, there is no upper bound to which the mixture may be heated, it is preferred that the mixture be heated to above the critical temperature but less than 230°C, more preferably less than 99°C.

The heating may be done by any method which raises the temperature of the mixture to the required temperatures in the time desired by the practitioner.

The separation of the lignin solids from the mixture can be done by any technique, which includes but is not limited to centrifugation, gravity settling, filtration, elutriation,

Because the process is generally done after hydrolysis, the mixture may contain at least one enzyme capable of converting cellulose to sugars. Many enzymes are known in the art and the techniques to evaluate whether such an enzyme is capable of converting cellulose to sugars is well established.

Since the mixture is generally derived from a biomass feedstock, the heating of the mixture is usually preceded by a steam explosion step conducted upon the biomass feedstock. Steam explosion of biomass, in particular cellulosic biomass, is well known in the art.

The point of separation of lignin can occur during many points during the conversion of a biomass feedstock. For example, the heating of the mixture and separation could be conducted after the biomass has been at least partially hydrolyzed to sugars and before the fermentation of the sugars to a final product.

The heating and separation can also occur after the at least partially hydrolyzed biomass has been converted to an alcohol, also known as the fermentation step.

The heating and separation of the lignin can also be conducted as part of a distillation step to separate an alcohol from the water of the mixture.

pH is also considered an effective parameter, therefore the heating and separation may be carried out when pH of the mixture is above 7.0, or at least above 3.5.

### Experimental

As shown in the following experiments, a mixture derived from a biomass feedstock by washing, steam exploding, hydrolyizing and fermenting, was heated to 80°C and then cooled, heated or maintained to be separated at the temperature indicated in Table I. The samples were centrifuged at 3000 rpm for the time indicated and at the temperature indicated.

There appeared to be three phases across the samples. The first phase is the liquid phase which was very clear, amber and distinct in all samples separated at 80°C and 95°C. In the sample separated at room temperature, nominally 23°C, there was no observed evident phase separation, and in fact the phases looked slightly inverted with the gray solid like material on top of a slightly darker liquid phase. The presence of the three phases is most evident in the sample centrifuged at 50°C at 4 and 8 minutes.

The measurement in Table I is the percent of the test tube which contained the visually observed phase as measured on the height of the total amount of material measured along the straight portion of the wall of the test tube. The efficiency of the process is seen as the amount of liquid without solids was approximately 80% for the material separated at 80°C and 95°C.

**TABLE I - HEIGHT OF LIQUID FRACTION AFTER SEPARATION**

| | 2 min, 3000 rpm | | | | 4 min, 3000 rpm | | |
|---|---|---|---|---|---|---|---|
| TEMP (°C) | Liquid | Mixture | Solids | | Liquid | Mixture | Solids |
| 20 | 10.7% | 89.3% | 0.0% | | 0.0% | 100.0% | 0.0% |
| 50 | 14.1% | 73.1% | 12.8% | | 27.3% | 57.1% | 15.6% |
| 80 | 78.5% | 0.0% | 21.5% | | 76.0% | 0.0% | 24.0% |
| 90 | 83.3% | 0.0% | 16.7% | | 81.4% | 0.0% | 18.6% |
| | | | | | | | |

| | 6 min, 3000 rpm | | | | 8 min, 3000 rpm | | |
|---|---|---|---|---|---|---|---|
| | Liquid | Mixture | Solids | | Liquid | Mixture | Solids |
| 20 | 0.0% | 100.0% | 0.0% | | 0.0% | 100.0% | 0.0% |
| 50 | 66.7% | 24.0% | 9.3% | | 68.5% | 19.2% | 12.3% |
| 80 | 83.3% | 0.0% | 16.7% | | 77.3% | 0.0% | 22.7% |
| 90 | 80.3% | 0.0% | 19.7% | | 80.0% | 0.0% | 20.0% |

**TABLE II - SOLID CONTENT OF THE LIQUID REMOVED**

| time [min] | RoomT | 50°C | 80°C | 95°C |
|---|---|---|---|---|
| 2 | 9.55% | 9.65% | 7.92% | 7.93% |
| 4 | 9.23% | 9.20% | 7.92% | 7.94% |
| 6 | 8.65% | 8.45% | 7.84% | 7.99% |
| 8 | 8.32% | 7.81% | 7.91% | 7.84% |

**TABLE III - HUMIDITY CONTENT OF THE SOLIDS**

| time | Room T | 50 °C | 80 °C | 95 °C |
|---|---|---|---|---|
| 2 | 72.94% | 70.14% | 68.86% | 67.82% |
| 8 | 72.76% | 70.04% | 67.72% | 64.47% |

**TABLE IV - RECAPTURE WEIGHT w/w%**

| time [min] | RoomT | 50°C | 80°C | 95°C |
|---|---|---|---|---|
| 2 | 55.74 | 59.19 | 62.18 | 64.23 |
| 4 | 54.78 | 59.51 | 65.53 | 68.60 |
| 6 | 62.36 | 61.95 | 61.46 | 61.64 |
| 8 | 60.48 | 59.74 | 59.14 | 59.88 |

**TABLE V - AVERAGE RECAPTURE RATE VERSUS TEMPERATURE**

| Temp (°C) | w/% |
|---|---|
| RoomT | 58.34 |
| 50 | 60.10 |
| 80 | 62.08 |
| 95 | 63.58 |

### VACUUM FILTRATION

In addition to the centrifuge, successful vacuum filtration was demonstrated

100 ml of the solution was filtered through a filtering area of 100 cm² under a vacuum of 0.5 bar: in the case in which the solution was filtered at 25 °C, the separation time was of

190 seconds and the drying time was of 90 seconds. In the case in which the solution is filtered at 55 °C, the separation time was of 45 seconds and the drying time was of 15 seconds.

150 ml of the mixture was filtered through filtering area of 100 cm² under a vacuum of 0.5 bar: in the case in which the solution was filtered at 25 °C, the separation time was of 420 seconds and the drying time was of 90 seconds. In the case in which the solution is filtered at 50 °C, the separation time was of 55 seconds and the drying time was of 15 seconds.

Also the final moisture of the cake was improved by the increasing of the temperature: in the case at 25 °C the cake moisture was of 64.4%wt; while in the case at 55 °C the cake moisture was of 59.16%.

## Claims

1. A method for separating lignin from an aqueous mixture having a pH above 3.5, comprising the steps of:
a) heating the mixture to a temperature above a critical temperature in the range of 45°C to 98°C,
b) separating the lignin solids from the mixture at a separation temperature which is at or above the critical temperature, wherein the mixture is derived from a biomass feedstock and the step of heating the mixture is preceded by a steam explosion step conducted upon the biomass feedstock.

2. The method of claim 1 wherein at least a portion of the separation is done by a centrifuge.

3. The method of claim 1, wherein at least a portion of the separation is done by filtration.

4. The method of claim 1, wherein at least a portion of the separation is done by gravity.

5. The method of any one of claims 1 to 4, wherein the mixture contains an enzyme capable of converting cellulose to sugars.

6. The method of any one of claims 1 to 5, wherein the heating of the mixture is conducted after the biomass has been at least partially hydrolyzed to sugars.

7. The method of claim 6, wherein the step of heating the mixture is conducted after the at least partially hydrolyzed biomass has been converted to an alcohol.

8. The method of any one of claims 1 to 7, wherein a portion of the step of heating the mixture is conducted as part of a distillation step to separate an alcohol from water.

9. The method of any of claims 1 to 8, wherein the pH of the mixture is above 7.0.

10. The method of any of claims 1 to 9, wherein the critical temperature is in a range having an upper temperature and a lower temperature and the upper temperature is 97°C and the lower temperature is 60°C.

11. The method of claim 10, wherein the lower temperature is 70°C and the upper temperature is 97°C.

12. The method of claim 10, wherein the lower temperature of the range is 74°C.

## Patentansprüche

1. Verfahren zum Abtrennen von Lignin aus einer wässrigen Mischung mit einem pH über 3,5, das die Schritte umfasst:
a) Erwärmen der Mischung auf eine Temperatur oberhalb einer kritischen Temperatur in dem Bereich von 45°C bis 98°C,
b) Abtrennen der Ligninfeststoffe aus der Mischung bei einer Abtrennungstemperatur, welche an oder oberhalb der kritischen Temperatur liegt, wobei die Mischung aus einem Biomasseausgangsmaterial abgeleitet ist und dem Schritt des Erwärmens der Mischung ein auf das Biomasseausgangsmaterial angewandter Dampfexplosionsschritt vorgeschaltet ist.

2. Verfahren nach Anspruch 1, wobei zumindest ein Teil der Abtrennung durch eine Zentrifuge vorgenommen wird.

3. Verfahren nach Anspruch 1, wobei zumindest ein Teil der Abtrennung durch Filtration vorgenommen wird.

4. Verfahren nach Anspruch 1, wobei zumindest ein Teil der Abtrennung durch Gravitation vorgenommen wird.

5. Verfahren nach einem der Ansprüche 1 bis 4, wobei die Mischung ein Enzym enthält, das in der Lage ist, Zellulose in Zucker umzuwandeln.

6. Verfahren nach einem der Ansprüche 1 bis 5, wobei das Erwärmen der Mischung durchgeführt wird, nachdem die Biomasse zumindest teilweise zu Zuckern hydrolysiert worden ist.

7. Verfahren nach Anspruch 6, wobei der Schritt des Erwärmens der Mischung durchgeführt wird, nachdem die zumindest teilweise hydrolysierte Biomasse in einen Alkohol umgewandelt worden ist.

8. Verfahren nach einem der Ansprüche 1 bis 7, wobei ein Teil des Schritts des Erwärmens der Mischung als ein Teil eines Destillationsschritts zum Abtrennen eines Alkohols von Wasser durchgeführt wird.

9. Verfahren nach einem der Ansprüche 1 bis 8, wobei der pH der Mischung über 7,0 liegt.

10. Verfahren nach einem der Ansprüche 1 bis 9, wobei die kritische Temperatur in einem Bereich liegt, der eine obere Temperatur und eine untere Temperatur aufweist, und die obere Temperatur 97°C ist und die untere Temperatur 60°C ist.

11. Verfahren nach Anspruch 10, wobei die untere Temperatur 70°C ist und die obere Temperatur 97°C ist.

12. Verfahren nach Anspruch 10, wobei die untere Temperatur des Bereichs 74°C ist.

## Revendications

1. Procédé pour séparer la lignine d'un mélange aqueux ayant un pH supérieur à 3,5, comprenant les étapes de :
a) chauffage du mélange à une température supérieure à une température critique dans la plage de 45°C à 98°C,
b) séparation des solides de la lignine du mélange à une température de séparation qui est à ou supérieure à la température critique, où le mélange est dérivé d'une charge de biomasse et l'étape de chauffage du mélange est précédée par une étape d'explosion à la vapeur conduite sur la charge de biomasse.

2. Procédé selon la revendication 1 où au moins une partie de la séparation est réalisée par une centrifugeuse.

3. Procédé selon la revendication 1, où au moins une partie de la séparation est réalisée par filtration.

4. Procédé selon la revendication 1, où au moins une partie de la séparation est réalisée par gravité.

5. Procédé selon l'une quelconque des revendications 1 à 4, où le mélange contient une enzyme capable de convertir la cellulose en sucres.

6. Procédé selon l'une quelconque des revendications 1 à 5, où le chauffage du mélange est conduit après que la biomasse a été au moins partiellement hydrolysée en sucres.

7. Procédé selon la revendication 6, où l'étape de chauffage du mélange est conduite après que la biomasse au moins partiellement hydrolysée a été convertie en un alcool.

8. Procédé selon l'une quelconque des revendications 1 à 7, où une partie de l'étape de chauffage du mélange est conduite dans le cadre d'une étape de distillation pour séparer un alcool de l'eau.

9. Procédé selon l'une quelconque des revendications 1 à 8, où le pH du mélange est supérieur à 7,0.

10. Procédé selon l'une quelconque des revendications 1 à 9, où la température critique est dans une plage ayant une température supérieure et une température inférieure et la température supérieure est 97°C et la température inférieure est 60°C.

11. Procédé selon la revendication 10, où la température inférieure est 70°C et la température supérieure est 97°C.

12. Procédé selon la revendication 10, où la température inférieure de la plage est 74°C.
